# EUROPÄISCHE PATENTSCHRIFT

(11) **EP 0 503 136 B1**
(45) Veröffentlichungstag und Bekanntmachung des Hinweises auf die Patenterteilung: **20.03.1996**
(21) Anmeldenummer: 91117406.8
(22) Anmeldetag: 11.10.1991
(51) Int. Cl.: A61F 2/16

(54) **Verfahren und Vorrichtung zum Falten einer gummielastischen Intraokularlinse**
Method and apparatus for folding a flexible intraocular lens
Procédé et dispositif pour plier une lentille intraoculaire souple

(30) Priorität: 14.03.1991 DE 4108303
(43) Veröffentlichungstag der Anmeldung: 16.09.1992
(73) Patentinhaber: Chiron Adatomed Pharmazeutische und Medizintechnische Gesellschaft mbH, D-85609 Dornach (DE)
(72) Erfinder: Kanert, Otmar, Prof.Dr., W-4600 Dortmund 50 (DE); Kammann, Jochen, Dr.med., W-4600 Dortmund (DE); Dretzler, Ulrich, Dipl.-Ing., W-4600 Dortmund 50 (DE)
(74) Vertreter: Nöth, Heinz, Dipl.-Phys.

(56) Entgegenhaltungen:
- EP-A- 270 257
- DE-C- 4 039 119
- US-A- 4 681 102
- US-A- 4 976 716

## Beschreibung

Die Erfindung betrifft ein Verfahren und eine Vorrichtung zum Falten einer gummielastischen Intraokularlinse.

Es ist bekannt, Intraokularlinsen aus gummielastischem Material, insbesondere Silikonlinsen, in gefaltetem Zustand bei einer Kataraktoperation in die natürliche, im Auge verbliebene Linsenkapsel einzusetzen. Hierbei gestaltet sich der Faltvorgang recht mühsam, da die Linse während des Faltens häufig zwischen den beiden Greiferelementen des Implantierwerkzeugs aufgrund seiner Gummielastizität herausspringt. Dies geschieht immer dann, wenn der gummielastische Linsenkörper zwischen den Greiferelementen des normalerweise als Pinzette ausgebildeten Implantierwerkzeugs nicht exakt positioniert ist.

Zur Behebung dieser Mängel schlägt die EP-A-0 270 257 eine Vorrichtung zum Einsetzen einer Intraokularlinse in die Linsenkapsel vor, die im wesentlichen aus einem langgezogenen Hohlrohr mit Ein- und Auslaßende und einer Kompressionseinrichtung, insbesondere Kolbenstange, sowie einer Schubeinrichtung, insbesondere Schubstange, besteht, wobei durch die Kompressionseinrichtung elastisches und als Intraokularlinse geeignetes Material in das Hohlrohrinnere gepreßt und die entsprechend dem Rohrinneren gestaltete Intraokularlinse mittels der Schubeinrichtung aus dem Rohrinneren ausgestoßen und in die Augenkapsel befördert wird.

Aus der DE-PS 33 48 066 ist schließlich eine chirurgische Vorrichtung zur Implantation verformbarer Intraokularlinsen in verschiedenen Ausführungsformen bekannt, bei der in einer Ausführungsform zwei gegeneinander bewegliche längliche Backen einen geschlossenen Hohlraum bilden, der zur Aufnahme der komprimierten Intraokularlinse dient. Die Intraokularlinse wird dabei in verschiedenster Form zwischen die länglichen Backen geklemmt, die Unterbringung erfolgt dabei willkürlich. Bei der Handhabung der Intraokularlinsen in dieser Form können Beschädigungen nicht ausgeschlossen werden. Ferner treten Probleme beim operativen Eingriff im Auge auf, da der Austritt der Linse aus der Vorrichtung relativ unkontrolliert, je nach der zufälligen Unterbringung zwischen den beiden Backen erfolgt. Durch die manuelle Einbringung der Linse in die Vorrichtung werden relativ hohe Anforderungen an die Geschicklichkeit des medizinischen Fachpersonals gestellt.

Bei einer anderen Ausführungsform, die in der genannten Patentschrift beschrieben wird, ist eine Kammer zur Aufnahme der unverformten Intraokularlinse vorhanden, und die Intraokularlinse kann mit Hilfe eines Druckmittels durch einen Auslaß mit sehr kleinem Querschnitt in das Auge gedrückt werden. Das Durchpressen durch den Auslaß erfolgt hierbei ebenfalls willkürlich, je nachdem wie sich die Linse in zufälliger Form vor den Auslaß setzt, wird sie sich undefiniert zusammenfalten. Dadurch kann natürlich ein definierter, gleichmäßiger Austritt in das Auge nicht erfolgen. Ebenfalls ist eine Beschädigung der empfindlichen Intraokularlinsen beim Durchpressen durch den engen Auslaß nicht ausgeschlossen.

Hier setzt die Erfindung ein, deren Aufgabe es ist, mit einem Verfahren und einer Vorrichtung gummielastische Intraokularlinsen mit mechanischen Mitteln, schonend, definiert gleichmäßig zu falten, so daß sie in einem Implantationswerkzeug exakt positioniert werden können.

Diese Aufgabe wird erfindungsgemäß durch ein Verfahren zum Falten einer gummielastischen Intraokularlinse gemäß Anspruch 1 und durch eine Vorrichtung gemäß Anspruch 2 zur Durchfuhrung dieses Verfahrens gelöst, wobei gemäß Anspruch 1 die Intraokularlinse z.B. mechanisch oder auch pneumatisch durch Ansaugen auf einer Auflageflache fixiert und anschließend mittels zweier gegenläufig und synchron auf die Intraokularlinse zu bewegter Zustellstemptel so gefaltet wird, daß die Intraokularlinse die geringfügig verkleinerte Innenkontur eines Röhrchens eines Implantationswerkzeuges nachbildet und nach Lösen der Linsenfixiereinrichtung die gefaltete Intraokularlinse mittels eines Einschubstempels in das Röhrcheninnere des Implantationswerkzeuges gedrückt wird.

Die zur Intraokularlinse weisenden Stirnflächen der Zustellstempel sind konkav abgerundet, so daß ihre Form etwa der Innenkontur des Röhrchens eines Implantationswerkzeugs entspricht. Dieses Röhrchen wird vor dem Faltvorgang an der Vorrichtung mit einer Klemmeinrichtung befestigt. Das Röhrchen liegt dabei orthogonal zur Zustellbewegung der beiden Zustellstempel. Durch die Ausformung der Stirnflächen der sich aufeinander zu bewegenden Zustellstempel bewegt sich die Haptik der Intraokularlinse entlang der ausgeformten Stirnflächen, und das Falten der Intraokularlinse beginnt. In Endstellung der Zustellstempel, also nach abgeschlossenem Faltvorgang, bilden die Auflagefläche und die Stirnflächen der Zustellstempel die Innenkontur des Röhrchens geringfügig verkleinert nach, und es kann mit einem Einführstempel die nun gefaltete Intraokularlinse in das Röhrcheninnere in einer Richtung senkrecht zum gefalteten Querschnitt geschoben werden.

In vorteilhafter Weise ist die Auflagefläche in dem Bereich, auf dem der optische Teil der Intraokularlinse aufliegt, konisch ausgeformt, z.B. mit einer Tiefe von etwa 0,3 mm. Dadurch wird die Positionierung der Linse in Zusammenwirken mit der Fixiereinrichtung verbessert. Bei der Verwendung einer mechanischen Fixiereinrichtung in Form eines Niederhalters bewirkt der Niederhalter, daß bei der Zustellbewegung der Zustellstempel sich der optische Teil der Intraokularlinse anhebt. Verwendet man dagegen pneumatische Fixiereinrichtungen, kann im Bereich der Auflagefläche mit einem geringen Unterdruck die Intraokularlinse an der Auflagefläche gehalten werden. Vor dem Einschieben der gefalteten Intraokularlinse in das Röhrcheninnere wird die Wirkung der Fixiereinrichtung durch Entfernung des mechanischen Niederhalters bzw. durch das Abschalten des Unterdrucks aufgehoben.

Für die Begrenzung der Bewegung eines mechanischen Niederhalters ist bevorzugt ein mechanischer Anschlag vorgesehen, so daß der Niederhalter in einer Stellung festgehalten wird, in der er auf dem optischen Teil der Intraokularlinse praktisch ohne Kraftwirkung aufliegt. Der Anschlag ist in Abhängigkeit von der Dicke der jeweiligen Linse einstellbar. Die Aufgabe des Niederhalters besteht darin, zu verhindern, daß sich beim Faltvorgang der optische Teil der Intraokularlinse von der Auflagefläche abhebt.

Je nach Form der Innenkontur des Röhrchens eines Implantationswerkzeuges kann die Endform der gefalteten Intraokularlinse von kreisrund bis flachelliptisch sein.

Das erfindungsgemäße Falten bewirkt eine äußerst schonende Behandlung der Intraokularlinse, da die Biegung des gummielastischen Materials zum größten Teil im nichtoptischen Teil der Intraokularlinse erfolgt und die Übergangsradien relativ großzügig bemessen sind.

Bevorzugt kommt die Faltvorrichtung bei Einbringen einer gefalteten Intraokularlinse in ein Implantationswerkzeug zum Einsatz, wie es in der deutschen Patentanmeldung P 40 30 492.2 beschrieben ist.

Günstig wirkt sich die Verwendung einer sterilen Flüssigkeit aus, die ein besseres Gleiten beim Faltvorgang und beim Einschieben in das Röhrcheninnere bewirkt.

Mit dem erfindungsgemäßen Verfahren und der dazu gehörigen Vorrichtung können beliebig viele Intraokularlinsen in eine definierte Form gefaltet werden. Es ist dadurch möglich, daß die Linsen immer angepaßt an den Röhrchenquerschnitt in das Röhrcheninnere eines Implantationswerkzeuges eingeschoben werden. Dadurch ergeben sich bei der Operation im Auge immer gleiche Entfaltungsverhalten der Introkularlinsen.

Nachfolgend soll die Erfindung anhand eines Ausführungsbeispiels näher erläutert werden.
- Fig. 1: zeigt einen Ausschnitt einer Seitenansicht des Ausführungsbeispiels mit eingelegter ungefalteter Intraokularlinse;
- Fig. 2: zeigt einen Ausschnitt der Vorderansicht des Ausführungsbeispiels;
- Fig. 3: zeigt die gleiche Ansicht nach vollendetem Faltvorgang.

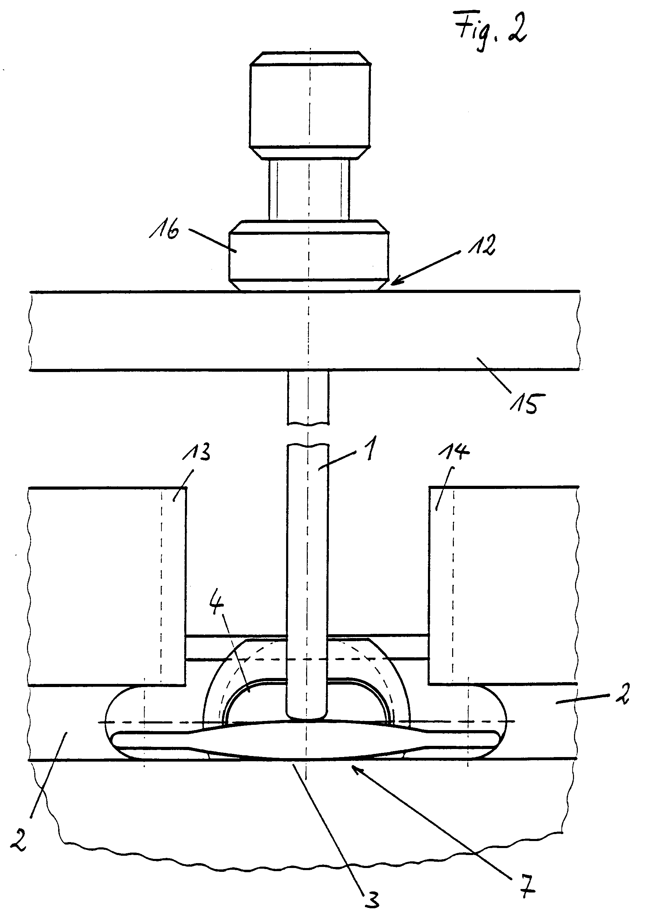

Nach dem Einlegen einer Intraokularlinse 7 auf eine Auflage 3 in der Vorrichtung wird ein Niederhalter 1 bis zum Erreichen eines mechanischen Anschlags 12 (Fig. 2) abgesetzt. Der Niederhalter 1 liegt dabei auf dem optischen Teil 9 der Linse 7 ohne Krafteinwirkung auf. Anschließend erfolgt die synchrone Zustellbewegung von Zustellstempeln 2. Nach Berühren der Stirnflächen der Zustellstempel 2 mit der Haptik 10 der Intraokularlinse 7 wird die Haptik an diesen gebogen, und der Faltvorgang beginnt. Der Niederhalter 1 verhindert dabei ein Anheben des optischen Teils 9 der Linse 7.

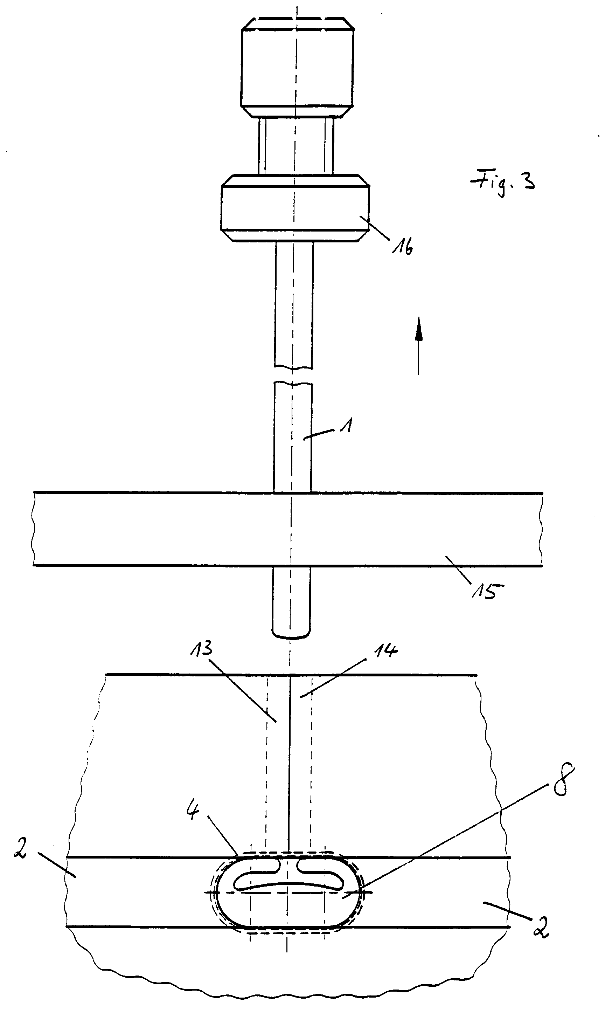

Nach Beendigung der Zustellbewegung bilden die Stirnflächen der Zustellstempel 2 und die Auflagefläche der Auflage 3 eine Kontur, die der Kontur des Röhrcheninneren eines Implantationswerkzeugs 4 entspricht und geringfügig kleiner ist als die Innenkontur des röhrchenförmigen Implantationswerkzeugs 4 (vgl. Fig. 3, gestrichelte Darstellung). In diesem Ausführungsbeispiel hat die Innenkontur eine ovale Form. Es ist aber auch eine annähernd kreisrunde Kontur möglich.

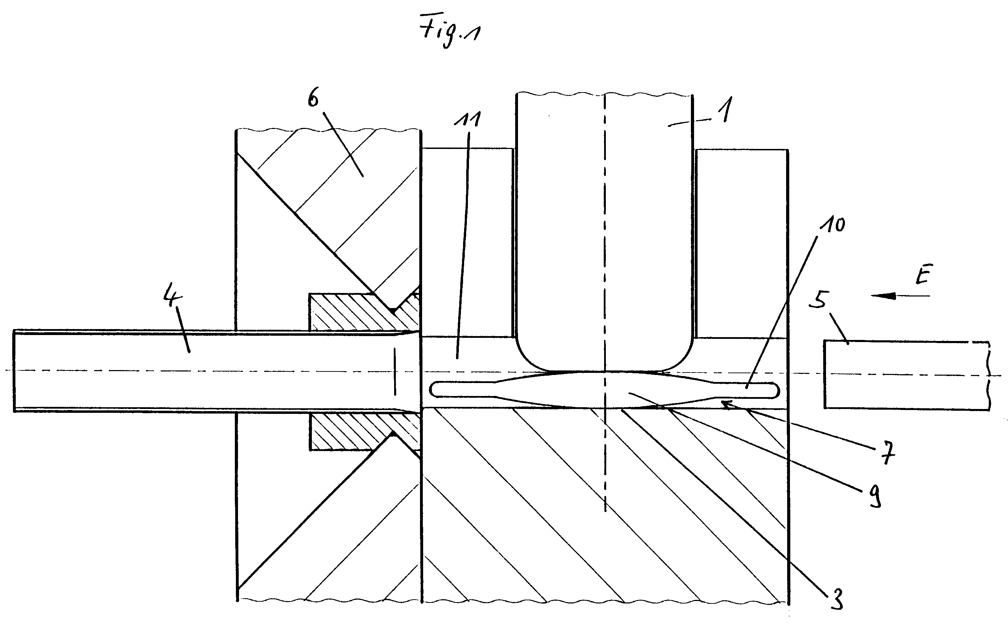

Die Zustellstempel 2 enthalten Ausnehmungen 13, 14, die dem Querschnitt des Niederhalters 1 angepaßt sind. Im Sinne des Faltvorganges ist es von Vorteil, den Querschnitt des Niederhalters 1 länglich auszuführen, wobei der Niederhalter in Einschubrichtung E (Fig. 1), d. h. senkrecht zur Faltrichtung (Fig. 2, 3), länglich ausgebildet ist.

Nach Abschluß des Faltvorgangs wird der Einschubstempel 5, dessen Bewegungsrichtung orthogonal zur Zustellbewegung der Zustellstempel 2 liegt, auf die gefaltete Intraokularlinse 8 zu bewegt, und diese wird in das Röhrcheninnere eines Implantationswerkzeugs 4 eingeschoben.

Vor Beginn des Faltvorgangs wird das Röhrchen des Implantationswerkzeugs 4 an der Vorrichtung mit einer Klemmeinrichtung 6 befestigt, so daß die Innenkontur des Röhrchens 4 mit einem Ausschubkanal 11, der von den beiden Zustellstempeln 2 gebildet wird, ausgerichtet ist. Dadurch kann die gefaltete Linse 8 ohne jegliche Beschädigung in das Röhrcheninnere des röhrchenförmigen Implantationswerkzeugs 4 mit dem Einschubstempel 5 geschoben werden. Die stets gleichförmig gefalteten Linsen 8 befinden sich daher immer definiert positioniert in gleicher Lage im Röhrchen des Implantationswerkzeugs 4 (Fig. 3).

Verbessert kann die Vorrichtung in einfacher Weise dadurch werden, daß in der Auflage 3 für den optischen Teil 9 der Linse 7 eine konische Ausnehmung mit einer Tiefe von etwa 0,3 mm vorgesehen wird. Dadurch kann eine bessere definierte Positionierung der Linse erfolgen. Auch wird in Zusammenwirkung mit dem Niederhalter 1 ein leichtes Anheben der Haptik 10 und somit eine verbesserte Vorbereitung des Faltvorgangs erzielt. Auch bei Verwendung einer mit Unterdruck wirkenden Fixiereinrichtung kann dieser Effekt erzielt werden.

Damit der Niederhalter 1 möglichst ohne Krafteinwirkung auf der zu faltenden Intraokularlinse 7 aufliegt, kann der Niederhalter 1 in seiner auf die Intraokularlinse 7 aufgesetzten Positionierung am bereits erwähnten Anschlag 12 anliegen. Beim dargestellten Ausführungsbeispiel (Figuren 2, 3) wird dieser Anschlag 12 von einer Schraubmutter 16, die am Niederhalter 1 verstellt werden kann, gebildet. Diese Schraubmutter 16 wirkt zur Bildung des Anschlages 12 mit einer Grundplatte 15 des Faltgerätes zusammen (Fig. 2). Je nach Dicke der zu faltenden Intraokularlinse 7 wird die Schraubmutter 16 am Niederhalter 1 verstellt. Hierdurch wird gewährleistet, daß auch bei unterschiedlichen Linsendicken der Niederhalter jeweils ohne Krafteinwirkung auf der zu faltenden Linse 7 aufliegt.

## Patentansprüche

1. Verfahren zum Falten einer gummielastischen Intraokularlinse,
dadurch **gekennzeichnet,**
daß eine Intraokularlinse (7) auf einer Auflagefläche (3) mittels einer lösbaren Linsenfixiereinrichtung fixiert und anschließend mittels zweier synchron gegeneinander bewegter Zustellstempel (2) so gefaltet wird, daß die Intraokularlinse (7), die geringfügig verkleinerte Innenkontur eines Röhrchens eines Implantationswerkzeuges (4) nachbildet und nach Lösen der Linsenfixiereinrichtung die gefaltete Intraokularlinse (8) mittels eines Einschubstempels (5) in das Röhrcheninnere des Implantationswerkzeugs (4) gedrückt wird.

2. Vorrichtung zum Falten einer gummielastischen Intraokularlinse zur Durchführung des Verfahrens nach Anspruch 1 und Röhrchen eines Implantationswerkzeugs, dadurch gekennzeichnet, daß sie eine Auflagefläche (3) für die Intraokularlinse (7) und zwei synchron entgegengesetzt zueinander sowie auf der Ebene der Auflagefläche (3) bewegbare Zustellstempel (2) aufweist, die im zusammengefahrenen Zustand an den die Intraokularlinse (8) berührenden Seiten die Innenkontur eines Röhrchens eines Implantationswerkzeuges (4) geringfügig verkleinert nachbilden, und daß ferner eine lösbare Linsenfixiereinrichtung, die die Intraokularlinse (7) auf der Auflagefläche (3) fixiert, sowie ein in Richtung des Röhrcheninneren des Implantationswerkzeugs (4) bewegbarer Einschubstempel (5) vorhanden sind.

3. Vorrichtung nach Anspruch 2, dadurch gekennzeichnet, daß die Auflagefläche (3) zur Aufnahme der Intraokularlinse (7) konisch ausgeformt ist.

4. Vorrichtung nach Anspruch 2 oder 3, dadurch gekennzeichnet, daß die Fixiereinrichtung ein gegen die Auflagefläche (3) bewegbarer Niederhalter (1) ist.

5. Vorrichtung nach Anspruch 2 oder 3, dadurch gekennzeichnet, daß die Fixiereinrichtung ein Unterdruckerzeuger ist, der den Unterdruck in der konischen Ausformung der Auflagefläche (3) wirken läßt.

6. Vorrichtung nach einem der Ansprüche 2 bis 4, dadurch gekennzeichnet, daß der Niederhalter (1) in seiner Fixierstellung an einem Anschlag (12) anliegt und die Intraokularlinse linse (7) ohne Krafteinwirkung berührt.

7. Vorrichtung nach einem der Ansprüche 2 bis 4 und 6, dadurch gekennzeichnet, daß der Niederhalter (1) in Einschubrichtung (E) des Einschubstempels (5) einen länglichen Querschnitt aufweist.

8. Vorrichtung nach einem der Ansprüche 2 bis 7, dadurch gekennzeichnet, daß die Einschubrichtung (E) des Einschubstempels (5) senkrecht zur Zustellbewegung der Zustellstempel (2) liegt.

## Claims

1. A method of folding a resilient intraocular lens, characterised in that an intraocular lens (7) is feed on a support surface (3) by means of a releasable lens fixing device and then folded by means of two feed pressing members (2) which are moved synchronously towards each other, so that the intraocular lens (7) reproduces the slightly reduced internal contour of a tube portion of an implantation instrument (4) and after release of the lens fixing device the folded intraocular lens (8) is pressed by means of an insertion pressing member (5) into the interior of the tube portion of the implantation instrument (4).

2. Apparatus for folding a resilient intraocular lens for carrying out the method according to claim 1 and a tube portion of an implantation instrument, characterised in that the apparatus has a support surface (3) for the intraocular lens (7) and two feed pressing members (2) which are movable synchronously in opposite relationship to each other and on the plane of the support surface (3) and which in the condition of being brought together, at the sides which touch the intraocular lens (8), reproduce in a slightly reduced fashion the internal contour of a tube portion of an implantation instrument (4), and that in addition there are provided a releasable lens fling means which fixes the intraocular lens (7) on the support surface (3) and an insertion pressing member (5) which is movable in the direction of the interior of the tube portion of the implantation instrument (4).

3. Apparatus according to claim 2 characterised in that the support surface (3) is recessed in a conical configuration to receive the intraocular lens (7).

4. Apparatus according to claim 2 or claim 3 characterised in that the fixing device is a hold-down means (1) which is movable towards the support surface (3).

5. Apparatus according to claim 2 or claim 3 characterised in that the fixing device is a means for producing a reduced pressure which causes the reduced pressure to act in the conical recess of the support surface (3).

6. Apparatus according to one of claims 2 to 4 characterised in that in its fixing position the hold-own means (1) bears against an abutment (12) and touches the intraocular lens (7) without applying a force.

7. Apparatus according to one of claims 2 to 4 and 6 characterised in that the hold-down means (1) is of an elongate cross-section in the direction of insertion (E) of the insertion pressing member (5).

8. Apparatus according to one of claims 2 to 7 characterised in that the direction of insertion (E) of the insertion pressing member (5) is perpendicular to the feed movement of the feed pressing members (2).

## Revendications

1. Procédé pour plier une lentille intra-oculaire à élasticité du type caoutchouc, caractérisé en ce qu'on fixe une lentille intra-oculaire (7) sur une surface d'appui (3) au moyen d'un agencement de fixation de lentille amovible et qu'on la plie ensuite au moyen de deux pistons d'avance (2) déplacés en synchronisme l'un vers l'autre, de telle sorte que la lentille intra-oculaire (7) reproduit le contour intérieur légèrement réduit d'un petit tube d'un outil d'implantation (4), et qu'après dégagement de l'agencement de fixation de lentille on pousse la lentille intra-oculaire pliée (8) à l'intérieur du petit tube de l'outil d'implantation au moyen d'un piston d'introduction (5).

2. Dispositif pour plier une lentille intra-oculaire à élasticité du type caoutchouc pour la mise en oeuvre du procédé selon la revendication 1 et petit tube d'un outil d'implantation, caractérisé en ce qu'il comporte une surface d'appui (3) pour la lentille intra-oculaire (7) et deux pistons d'avance (2) déplaçables en synchronisme en sens inverse l'un de l'autre ainsi que sur le plan de la surface d'appui (3), qui dans l'état de contact mutuel reproduisent sur leur face en contact avec la lentille intra-oculaire (8), avec une légère réduction, le contour intérieur d'un petit tube d'un outil d'implantation (4), et que sont en outre prévus un agencement de fixation de lentille amovible, qui fixe la lentille intra-oculaire (7) sur la surface d'appui (3), ainsi qu'un piston d'introduction (5) déplaçable en direction de l'intérieur du petit tube de l'outil d'implantation (4).

3. Dispositif selon la revendication 2, caractérisé en ce que la surface d'appui (3) est conformée en cône pour la réception de la lentille intra-oculaire (7).

4. Dispositif selon l'une des revendications 2 et 3, caractérisé en ce que l'agencement de fixation est un serreflan (1) déplaçable vers la surface d'appui (3).

5. Dispositif selon l'une des revendications 2 et 3, caractérisé en ce que l'agencement de fixation est un générateur de dépression, que fait agir la dépression dans la conformation conique de la surface d'appui (3).

6. Dispositif selon l'une des revendications 2 à 4, caractérisé en ce que, dans sa position de fixation, le serre-flan (1) s'appuie sur une butée (12) et vient en contact avec la lentille intra-oculaire (7) sans exercer d'effort.

7. Dispositif selon l'une des revendications 2 à 4 et 6, caractérisé en ce que le serre-flan (1) présente une section transversale allongée dans la direction d'introduction (E) du piston d'introduction (5).

8. Dispositif selon l'une des revendications 2 à 7, caractérisé en ce que la direction d'introduction (E) du piston d'introduction (5) est perpendiculaire au mouvement d'avance des pistons d'avance (2).
